# EUROPEAN PATENT SPECIFICATION

(11) **EP 4 479 389 B1**
(45) Date of publication and mention of the grant of the patent: **04.11.2026**
(21) Application number: 23757121.1
(22) Date of filing: 17.02.2023
(51) Int. Cl.: C09K 9/02, C07D 241/48, G02F 1/1516, H01M 10/0568, H01M 4/02, H01M 4/60, G02F 1/1503

(54) **ELECTROCHEMICAL COMPOUNDS FOR LOW CURRENT ELECTROCHROMIC DEVICES**
ELEKTROCHEMISCHE VERBINDUNGEN FÜR ELEKTROCHROME NIEDERSTROM-VORRICHTUNGEN
COMPOSÉS ÉLECTROCHIMIQUES POUR DISPOSITIFS ÉLECTROCHROMIQUES À FAIBLE COURANT

(30) Priority: 18.02.2022 US 202263311694 P
(43) Date of publication of application: 25.12.2024
(73) Proprietor: GENTEX CORPORATION, Zeeland, Michigan 49464 (US)
(72) Inventor: GIRI, Punam, Zeeland, Michigan 49464 (US); KLOEPPNER, Leroy, Zeeland, Michigan 49464 (US); BAUMANN, Kelly, Zeeland, Michigan 49464 (US)
(74) Representative: Müller-Boré & Partner Patentanwälte PartG mbB
(86) International application number: PCT/US2023/062817
(87) International publication number: WO 2023/159179

(56) References cited:
- WO-A1-2021/064578
- US-A1- 2006 103 911
- US-A1- 2006 103 911
- US-A1- 2020 050 070
- US-A1- 2020 409 225
- US-A1- 2021 240 047
- US-A1- 2021 240 047
- US-A1- 2021 253 540
- US-A1- 2022 048 869

## Description

### FIELD

The present technology is generally related to redox compounds that are suitable for use in electrochromic devices having a persistent color memory and can provide a current during clearing for a substantial time period after being charged.

### BACKGROUND

Electrochromic devices have been well known for many years. When a sufficient electrical potential is applied across a pair of electrodes, an electrochromic medium, disposed between the electrodes, may become activated, changing its color and/or light transmissivity. Taking advantage of this, devices such as dimmable mirrors and windows have become increasingly popular in industries such as automotive and aviation. US 2021/240047 A1 describes electrochromic compounds and US 2006/103911 A1 discloses electrochromic compounds and associated media and devices.

This disclosure addresses phenazine electrochromic materials having improved weathering stability properties for use in electrochromic devices, in particular as the anodic redox species attached or confined to a polymer matrix film.

### SUMMARY

The present invention is as set out in the appended claims.

### BRIEF DESCRIPTION OF THE DRAWINGS

FIG. 1 shows the UV cycling durability (3 hours on/ 1 hour off) for Device A and Device B as described in the Examples.
FIG. 2 shows the 85°C cycling durability (3 hours on/ 1 hour off) for Device A and Device B as described in the Examples.

### DETAILED DESCRIPTION

Various embodiments are described hereinafter. It should be noted that the specific embodiments are not intended as an exhaustive description or as a limitation to the broader aspects discussed herein. One aspect described in conjunction with a particular embodiment is not necessarily limited to that embodiment and may be practiced with any other embodiment(s).

As used herein, "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 10% of the particular term.

The use of the terms "a" and "an" and "the" and similar referents in the context of describing the elements (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein may be performed in any suitable order unless otherwise indicated herein or otherwise clearly contradicted by context. The use of any and all examples, or exemplary language (*e.g*., "such as") provided herein, is intended merely to better illuminate the embodiments and does not pose a limitation on the scope of the claims unless otherwise stated. No language in the specification should be construed as indicating any non-claimed element as essential.

In general, "substituted" refers to an alkyl, alkenyl, aryl, or ether group, as defined below (e.g., an alkyl group) in which one or more bonds to a hydrogen atom contained therein are replaced by a bond to non-hydrogen or non-carbon atoms. Substituted groups also include groups in which one or more bonds to a carbon(s) or hydrogen(s) atom are replaced by one or more bonds, including double or triple bonds, to a heteroatom. Thus, a substituted group will be substituted with one or more substituents, unless otherwise specified. In some embodiments, a substituted group is substituted with 1, 2, 3, 4, 5, or 6 substituents. Examples of substituent groups include: halogens (i.e., F, Cl, Br, and I); hydroxyls; alkoxy, alkenoxy, alkynoxy, aryloxy, aralkyloxy, heterocyclyloxy, and heterocyclylalkoxy groups; carbonyls (oxo); carboxyls; esters; urethanes; oximes; hydroxylamines; alkoxyamines; aralkoxyamines; thiols; sulfides; sulfoxides; sulfones; sulfonyls; sulfonamides; amines; N-oxides; hydrazines; hydrazides; hydrazones; azides; amides; ureas; amidines; guanidines; enamines; imides; isocyanates; isothiocyanates; cyanates; thiocyanates; imines; nitro groups; nitriles (i.e., CN); and the like.

As used herein, "alkyl" groups include straight chain and branched alkyl groups having from 1 to about 20 carbon atoms, and typically from 1 to 12 carbons or, in some embodiments, from 1 to 8 carbon atoms. As employed herein, "alkyl groups" include cycloalkyl groups as defined below. Alkyl groups may be substituted or unsubstituted. An alkyl group may be substituted one or more times. An alkyl group may be substituted two or more times. Examples of straight chain alkyl groups include methyl, ethyl, n-propyl, n-butyl, n-pentyl, n-hexyl, n-heptyl, and n-octyl groups. Examples of branched alkyl groups include, but are not limited to, isopropyl, sec-butyl, t-butyl, neopentyl, isopentyl groups, and 1-cyclopentyl-4-methylpentyl. Representative substituted alkyl groups may be substituted one or more times with, for example, amino, ammonium, thio, hydroxy, cyano, alkoxy, and/or halo groups such as F, Cl, Br, and I groups. As used herein the term haloalkyl is an alkyl group having one or more halo groups. In some embodiments, haloalkyl refers to a per-haloalkyl group.

Cycloalkyl groups are cyclic alkyl groups such as, but not limited to, cyclopropyl, cyclobutyl, cyclopentyl, cyclohexyl, cycloheptyl, and cyclooctyl groups. In some embodiments, the cycloalkyl group has 3 to 8 ring members, whereas in other embodiments the number of ring carbon atoms range from 3 to 5, 6, or 7. Cycloalkyl groups may be substituted or unsubstituted. Cycloalkyl groups further include polycyclic cycloalkyl groups such as, but not limited to, norbornyl, adamantyl, bornyl, camphenyl, isocamphenyl, and carenyl groups, and fused rings such as, but not limited to, decalinyl, and the like. Cycloalkyl groups also include rings that are substituted with straight or branched chain alkyl groups as defined above. Representative substituted cycloalkyl groups may be mono-substituted or substituted more than once, such as, but not limited to: 2,2-; 2,3-; 2,4-; 2,5-; or 2,6-disubstituted cyclohexyl groups or mono-, di-, or trisubstituted norbornyl or cycloheptyl groups, which may be substituted with, for example, alkyl, alkoxy, amino, thio, hydroxy, cyano, and/or halo groups.

Alkenyl groups are straight chain, branched or cyclic alkyl groups having 2 to about 20 carbon atoms, and further including at least one double bond. In some embodiments alkenyl groups have from 1 to 12 carbons, or, typically, from 1 to 8 carbon atoms. Alkenyl groups may be substituted or unsubstituted. Alkenyl groups include, for instance, vinyl, propenyl, 2-butenyl, 3-butenyl, isobutenyl, cyclohexenyl, cyclopentenyl, cyclohexadienyl, butadienyl, pentadienyl, and hexadienyl groups among others. Alkenyl groups may be substituted similarly to alkyl groups. Divalent alkenyl groups, i.e., alkenyl groups with two points of attachment, include, but are not limited to, CH-CH=CH₂, C=CH₂, or C=CHCH₃.

As used herein, "aryl", or "aromatic," groups are cyclic aromatic hydrocarbons that do not contain heteroatoms. Aryl groups include monocyclic, bicyclic, and polycyclic ring systems. Thus, aryl groups include, but are not limited to, phenyl, azulenyl, heptalenyl, biphenylenyl, indacenyl, fluorenyl, phenanthrenyl, triphenylenyl, pyrenyl, naphthacenyl, chrysenyl, biphenyl, anthracenyl, indenyl, indanyl, pentalenyl, and naphthyl groups. An aryl group with one or more alkyl groups may also be referred to as alkaryl groups. In some embodiments, aryl groups contain 6-14 carbons, and in others from 6 to 12 or even 6-10 carbon atoms in the ring portions of the groups. The phrase "aryl groups" includes groups containing fused rings, such as fused aromatic-aliphatic ring systems (e.g., indanyl, tetrahydronaphthyl, and the like). Aryl groups may be substituted or unsubstituted.

Heterocyclyl or heterocycle refers to both aromatic and nonaromatic ring compounds including monocyclic, bicyclic, and polycyclic ring compounds containing 3 or more ring members of which one or more is a heteroatom such as, but not limited to, N, O, and S. Examples of heterocyclyl groups include, but are not limited to: unsaturated 3 to 8 membered rings containing 1 to 4 nitrogen atoms such as, but not limited to pyrrolyl, pyrrolinyl, imidazolyl, pyrazolyl, pyridinyl, dihydropyridinyl, pyrimidinyl, pyrazinyl, pyridazinyl, triazolyl (*e.g*. 4H-1,2,4-triazolyl, 1H-1,2,3-triazolyl, 2H-1,2,3-triazolyl etc.), tetrazolyl, (*e.g*. 1H-tetrazolyl, 2H tetrazolyl, etc.); saturated 3 to 8 membered rings containing 1 to 4 nitrogen atoms such as, but not limited to, pyrrolidinyl, imidazolidinyl, piperidinyl, piperazinyl; condensed unsaturated heterocyclic groups containing 1 to 4 nitrogen atoms such as, but not limited to, indolyl, isoindolyl, indolinyl, indolizinyl, benzimidazolyl, quinolyl, isoquinolyl, indazolyl, benzotriazolyl; unsaturated 3 to 8 membered rings containing 1 to 2 oxygen atoms and 1 to 3 nitrogen atoms such as, but not limited to, oxazolyl, isoxazolyl, oxadiazolyl (e.g. 1,2,4-oxadiazolyl, 1,3,4-oxadiazolyl, 1,2,5-oxadiazolyl, etc.); saturated 3 to 8 membered rings containing 1 to 2 oxygen atoms and 1 to 3 nitrogen atoms such as, but not limited to, morpholinyl; unsaturated condensed heterocyclic groups containing 1 to 2 oxygen atoms and 1 to 3 nitrogen atoms, for example, benzoxazolyl, benzoxadiazolyl, benzoxazinyl (*e.g*. 2H-1,4-benzoxazinyl etc.); unsaturated 3 to 8 membered rings containing 1 to 3 sulfur atoms and 1 to 3 nitrogen atoms such as, but not limited to, thiazolyl, isothiazolyl, thiadiazolyl (e.g. 1,2,3-thiadiazolyl, 1,2,4-thiadiazolyl, 1,3,4-thiadiazolyl, 1,2,5-thiadiazolyl, etc.); saturated 3 to 8 membered rings containing 1 to 2 sulfur atoms and 1 to 3 nitrogen atoms such as, but not limited to, thiazolodinyl; saturated and unsaturated 3 to 8 membered rings containing 1 to 2 sulfur atoms such as, but not limited to, thienyl, dihydrodithiinyl, dihydrodithionyl, tetrahydrothiophene, tetrahydrothiopyran; unsaturated condensed heterocyclic rings containing 1 to 2 sulfur atoms and 1 to 3 nitrogen atoms such as, but not limited to, benzothiazolyl, benzothiadiazolyl, benzothiazinyl (e.g. 2H-1,4-benzothiazinyl, etc.), dihydrobenzothiazinyl (*e.g*. 2H-3,4-dihydrobenzothiazinyl, etc.), unsaturated 3 to 8 membered rings containing oxygen atoms such as, but not limited to furyl; unsaturated condensed heterocyclic rings containing 1 to 2 oxygen atoms such as benzodioxolyl (*e.g*., 1,3-benzodioxoyl, etc.); unsaturated 3 to 8 membered rings containing an oxygen atom and 1 to 2 sulfur atoms such as, but not limited to, dihydrooxathiinyl; saturated 3 to 8 membered rings containing 1 to 2 oxygen atoms and 1 to 2 sulfur atoms such as 1,4-oxathiane; unsaturated condensed rings containing 1 to 2 sulfur atoms such as benzothienyl, benzodithiinyl; and unsaturated condensed heterocyclic rings containing an oxygen atom and 1 to 2 oxygen atoms such as benzoxathiinyl. Heterocyclyl group also include those described above in which one or more S atoms in the ring is double-bonded to one or two oxygen atoms (sulfoxides and sulfones). For example, heterocyclyl groups include tetrahydrothiophene oxide and tetrahydrothiophene 1,1-dioxide. Typical heterocyclyl groups contain 5 or 6 ring members. Thus, for example, heterocyclyl groups include morpholinyl, piperazinyl, piperidinyl, pyrrolidinyl, imidazolyl, pyrazolyl, 1,2,3-triazolyl, 1,2,4-triazolyl, tetrazolyl, thiophenyl, thiomorpholinyl, thiomorpholinyl in which the S atom of the thiomorpholinyl is bonded to one or more O atoms, pyrrolyl, pyridinyl homopiperazinyl, oxazolidin-2-onyl, pyrrolidin-2-onyl, oxazolyl, quinuclidinyl, thiazolyl, isoxazolyl, furanyl, dibenzylfuranyl, and tetrahydrofuranyl. Heterocyclyl or heterocycles may be substituted.

Heteroaryl groups are aromatic ring compounds containing 5 or more ring members, of which, one or more is a heteroatom such as, but not limited to, N, O, and S. Heteroaryl groups include, but are not limited to, groups such as pyrrolyl, pyrazolyl, triazolyl, tetrazolyl, oxazolyl, isoxazolyl, thiazolyl, pyridinyl, pyridazinyl, pyrimidinyl, pyrazinyl, thiophenyl, benzothiophenyl, furanyl, benzofuranyl, dibenzofuranyl, indolyl, azaindolyl (pyrrolopyridinyl), indazolyl, benzimidazolyl, imidazopyridinyl (azabenzimidazolyl), pyrazolopyridinyl, triazolopyridinyl, benzotriazolyl, benzoxazolyl, benzothiazolyl, benzothiadiazolyl, imidazopyridinyl, isoxazolopyridinyl, thianaphthyl, purinyl, xanthinyl, adeninyl, guaninyl, quinolinyl, isoquinolinyl, tetrahydroquinolinyl, quinoxalinyl, and quinazolinyl groups. Heteroaryl groups include fused ring compounds in which all rings are aromatic such as indolyl groups and include fused ring compounds in which only one of the rings is aromatic, such as 2,3-dihydro indolyl groups. Although the phrase "heteroaryl groups" includes fused ring compounds, the phrase does not include heteroaryl groups that have other groups bonded to one of the ring members, such as alkyl groups. Rather, heteroaryl groups with such substitution are referred to as "substituted heteroaryl groups." Representative substituted heteroaryl groups may be substituted one or more times with various substituents such as those listed above.

As used herein, the prefix "halo" refers to a halogen (*i.e.* F, Cl, Br, or I) being attached to the group being modified by the "halo" prefix. For example, haloaryls are halogenated aryl groups.

Groups described herein having two or more points of attachment (i.e., divalent, trivalent, or polyvalent) within the compound of the present technology are designated by use of the suffix, "ene." For example, divalent alkyl groups are alkylene groups, divalent aryl groups are arylene groups, divalent heteroaryl groups are divalent heteroarylene groups, and so forth.

The present disclosure relates to electrochromic devices that incorporate specific phenazine compounds as anodic redox species. As described below, the phenazine compounds disclosed herein are substituted with at least one sterically hindered group (*e.g.,* a tertiary butyl group). As demonstrated in the Examples, the phenazine compounds described herein are thermally stable and have improved weathering durability, for example, electrical cycling while under UV exposure (at about 390nm or greater). The phenazines described herein are electrochromic.

More broadly, disclosed herein are energy storage devices and/or electrochromic memory devices that include a cathodic material, and an anodic material such that when the cathodic material and anodic material are in an activated state, the devices maintains an activated (i.e. darkened) state for long periods of time at open circuit. When the terms cathodic and anodic material are used herein, at least one of them is electrochromic. The cathodic material/moiety may be associated with a first conductive substrate, or sequestered within a polymer matrix, and the anodic material/moiety may be associated with a second conductive substrate or sequestered within a polymeric matrix. For example, the anodic material may be covalently crosslinked and coated on a second conductive substrate and the cathodic material may be covalently crosslinked and coated on the surface of a first conductive substrate. By coating the anodic and cathodic materials to the substrate, the anodic and cathodic materials are prevented from migration within the device, thus potentially maintaining an activated stated indefinitely.

As noted above, the anodic or cathodic materials may also be part of the polymeric matrix with the anodic or cathodic material being covalently bound to the polymer. This may be accomplished with the presence of a functional group on the anodic or cathodic material that is reacted with the polymer or monomers that make up the polymer. For example, where the anodic or cathodic materials contain a hydroxyl group, the anodic or cathodic material may be bound into a polymer matrix via a condensation reaction or react with isocyanate functionality to form a polyurethane-based polymer matrix. Amines may also react with isocyanate functionalities to form urea and biuret linkages. It can be also anticipated that other cross-linked polymeric matrix can be formed using a multifunctional epoxy or polymers in combination with a curing agent like an amine, alcohol or anhydride or through base or acid catalyzed homopolymerization.

Typical solution-phase electrochromic devices contain at least one anodic (oxidizable) material or species, at least one cathodic (reducible) material or species, and a solvent. Upon applying a sufficient electrical potential to a solution-phase electrochromic device, *i.e.* activation, of a typical solution-phase devices will undergo a color change (i.e. darkened, or low transmission state). Because the anodic and cathodic materials are capable of free diffusion through the solvent while in the activated state, the electrochromic device will self-erase upon removal of the charging potential. It has now been found that sequestration of an anodic material on the surface of a conductive substrate while sequestrating the cathodic material on the surface of a second conductive substrate results in electrochromic devices that are configured to maintain the charged, *i.e.* activated, state for prolonged periods of time in comparison to typical solution-phase electrochromic devices, as long as the electrochromic anodic and cathodic films are separated by a ionic conductive electrolyte.

The electrochromic devices described herein may include at least one chamber defined by a first substrate having a first conductive surface, a second substrate having a second conductive surface, and a sealing member joining the first substrate to the second substrate with the first and second conductive surfaces contacting the sealing member. For the surface confinement of the anodic and cathodic materials, the anodic material may be sequestered on one conductive surface and the cathodic material may be sequestered on the other conductive surface. Within the chamber is disposed an electrolyte between the anodic and cathodic layers. The first and second substrates may be off-set to one another to allow for electric contact to be made with the first and second conductive surfaces, as is well established for other solution phase electrochromic devices.

In one aspect, an energy storage or memory device is provided. The energy storage or memory device may include a cathodic material and an anodic material. The cathodic material may be a viologen and the anodic material may be a phenazine as described herein.

In some embodiments, the anodic material comprises a phenazine compound represented as: 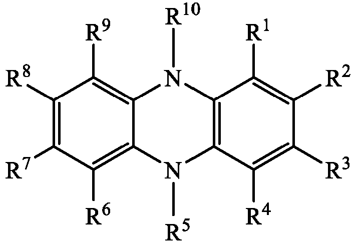 wherein:
R¹, R², R³, R⁴, R⁶, R⁷, R⁸, and R⁹ are each independently H, F, Cl, Br, I, CF₃, CN, OR¹¹, SR¹¹, NO₂, alkyl, alkoxy, aryl, heteroaryl,
amino, ammonium, or any two adjacent groups of R³, R⁴, R⁸, and
R⁹ may join to form a monocyclic, polycyclic, or heterocyclic group;
each R¹¹ is independently H or alkyl;
R⁵ and R¹⁰ are independently represented by:

   -L₁-A-L₂-OR¹⁴;

   each L₁ is independently an C₁-C₈ alkylene group;
   each L₂ is independently an C₁-C₈ alkylene group;
   each R¹⁴ is H or alkyl;
   A is an ammonium group represented by -N(R¹⁵)₂⁺-;
   each R¹⁵ is alkyl or alkylhydroxy; and
at least one of R², R³, R⁷, and R⁸ is a sterically hindered group.

A sterically hindered group may be selected from tertiary butyl, isobutyl, isopropyl, 2-ethyl butyl, 2-ethyl hexyl, sec-butyl, isopentyl, and neopentyl. A sterically hindered group may be an alkyl group comprising an isopropyl moiety. In some embodiments, a sterically hindered group is not a methyl group.

In some embodiments, R¹, R³, R⁴, R⁶, R⁸, and R⁹ are each independently H. In some embodiments, R¹, R³, R⁴, R⁶, R⁷, and R⁹ are each independently H.

In some embodiments, at least one of R², R³, R⁷, and R⁸ are independently C₂-C₈ alkyl. In some embodiments, at least one of R², R³, R⁷, and R⁸ are independently ethyl, propyl, isopropyl, butyl, isobutyl, sec-butyl, tertiary butyl, pentyl, neopentyl, hexyl, heptyl, or octyl. In some embodiments, at least one of R², R³, R⁷, and R⁸ are independently isopropyl, neopentyl or tertiary butyl.

In some embodiments, R² and R⁷ or R² and R⁸ are each independently C₂-C₈ alkyl. In some embodiments, R² and R⁷ or R² and R⁸ are each independently ethyl, propyl, isopropyl, butyl, isobutyl, sec-butyl, tertiary butyl, pentyl, neopentyl, hexyl, heptyl, or octyl. In some embodiments, wherein R² and R⁷ or R² and R⁸ are each independently isopropyl, neopentyl or tertiary butyl.

In some embodiments, R³ and R⁸ or R³ and R⁷ are each independently C₂-C₈ alkyl. In some embodiments, R³ and R⁸ or R³ and R⁷ are each independently ethyl, propyl, isopropyl, butyl, isobutyl, sec-butyl, tertiary butyl, pentyl, neopentyl, hexyl, heptyl, or octyl. In some embodiments, R³ and R⁸ or R³ and R⁷ are each independently isopropyl, neopentyl, or tertiary butyl.

In some embodiments, at least one of R², R³, R⁷, and R⁸ are independently aryl. In some embodiments, at least one of R², R³, R⁷, and R⁸ are independently phenyl.

In some embodiments, R² and R⁷ or R² and R⁸ are each independently aryl. In some embodiments, R² and R⁷ are each independently phenyl.

In some embodiments, R³ and R⁸ or R³ and R⁷ are each independently aryl. In some embodiments, R³ and R⁸ or R³ and R⁷ are each independently phenyl.

In some embodiments, at least one of R⁵ and R¹⁰ are independently represented by:

-L₁-A-L₂-OR¹⁴;

each L₁ is independently an C₁, C₂, C₃, C₄, C₅, C₆, C₇, or C₈ alkylene group;
each L₂ is independently an C₁, C₂, C₃, C₄, C₅, C₆, C₇, or C₈ alkylene group;
each R¹⁴ is H or alkyl;
A is an ammonium group represented by -N(R¹⁵ )₂⁺-; and
each R¹⁵ is C₁-C₈ alkyl or C₁-C₈ alkylhydroxyl.

In some embodiments,
each L₁ is independently an C₂ or C₄ alkylene group;
each L₂ is independently an C₃ alkylene group;
each R¹⁴ is H;
A is an ammonium group represented by -N(R¹⁵)₂⁺-; and
each R¹⁵ is C₁ alkyl or C₂ alkylhydroxyl.

In some embodiments, at least one of R⁵ and R¹⁰ are independently represented by: 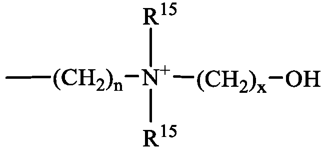
n is 1, 2, 3, 4, 5, 6, 7, or 8;
x is 1, 2, 3, 4, 5, 6, 7, or 8; and
each R¹⁵ is independently C₁-C₈ alkyl or C₁-C₈ alkylhydroxyl.

In some embodiments, n is 2 or 4;
x is 3; and
each R¹⁵ is independently C₁ alkyl or C₂ alkylhydroxyl.

In some embodiments, the compound is ammonium salt with an anion comprising F⁻, Cl⁻, Br⁻, I⁻, BF₄⁻, PF₆⁻, SbF₆⁻, AsF₆⁻, ClO₄⁻, SO₃CF₃⁻, N(CF₃SO₂)₂⁻, C(CF₃SO₂)₃⁻, N(SO₂C₂F₅)₂⁻, Al(OC(CF₃)₃)₄⁻, BAr₄⁻, or mixtures of anions thereof, wherein Ar is an aryl or fluorinated aryl group. In some embodiments, the anion is selected from PF₆⁻, SO₃CF₃⁻, and a mixture of anions thereof.

According to various embodiments herein, the phenazine compounds disclosed herein may be any one of the following compounds. As noted, some of the phenazines disclosed has at least one net, positive charge that is electronically balanced by an anion (not shown). The anion may be any of a variety of anions as noted above.

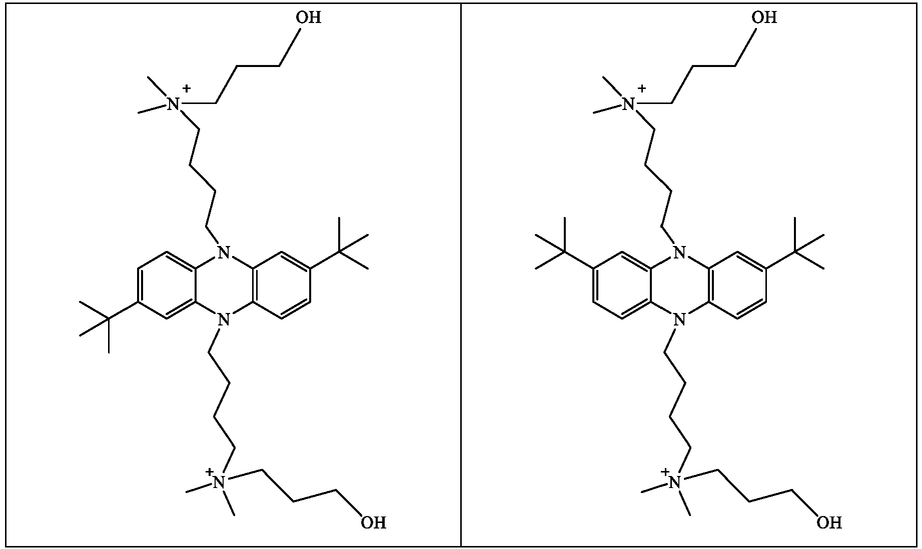

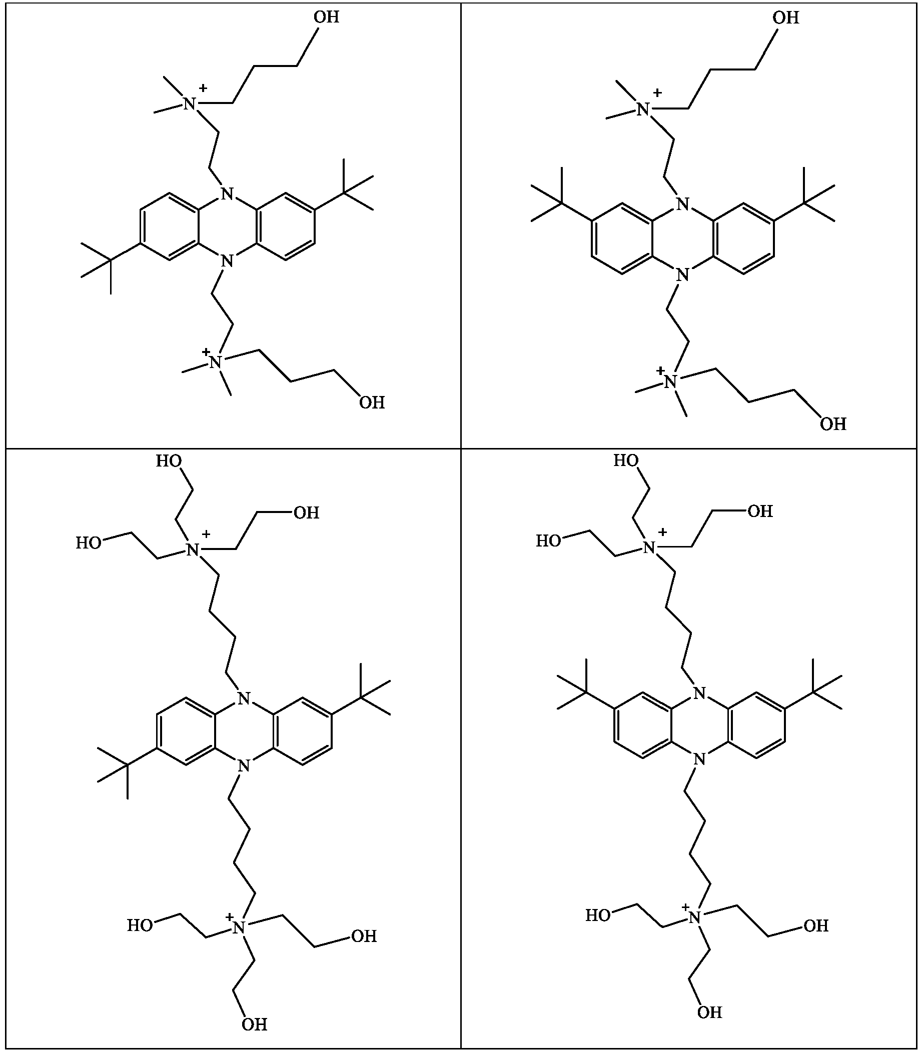

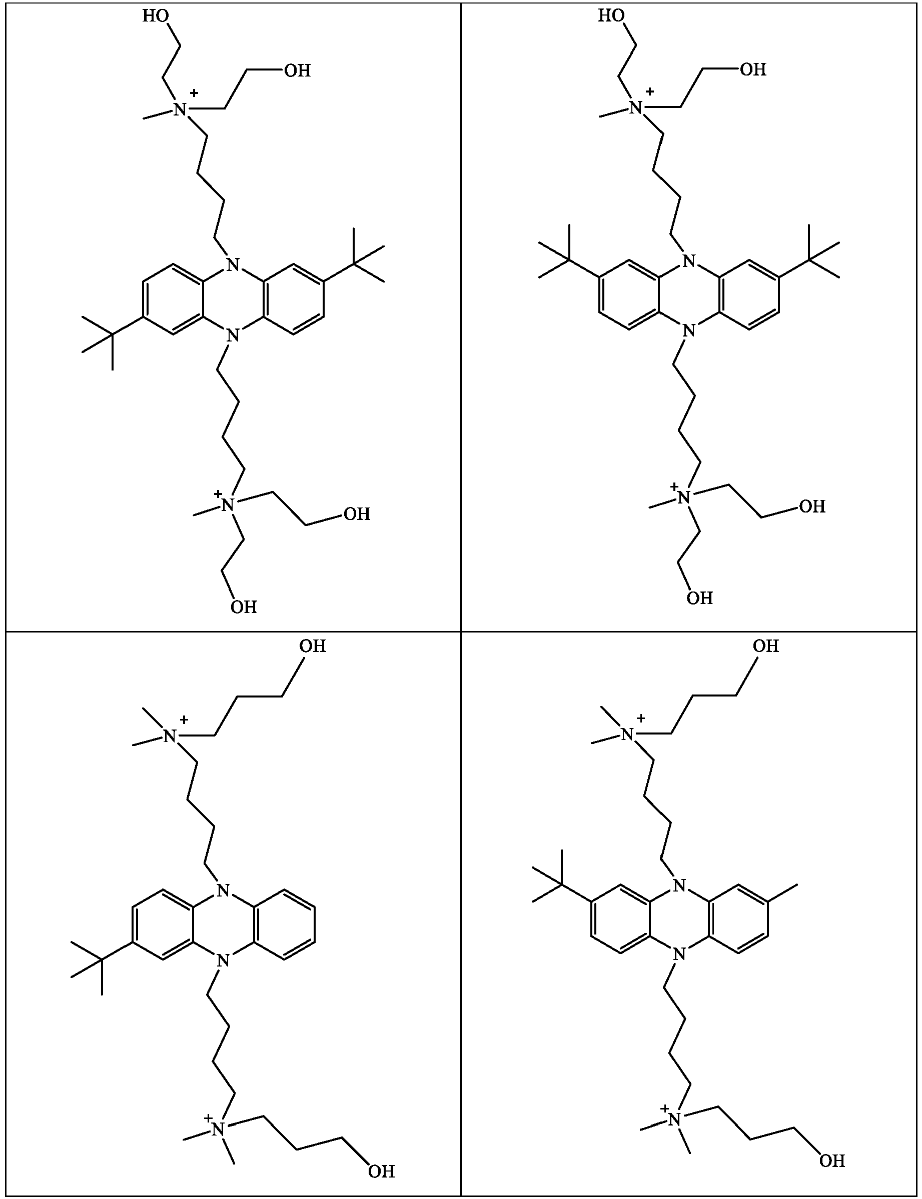

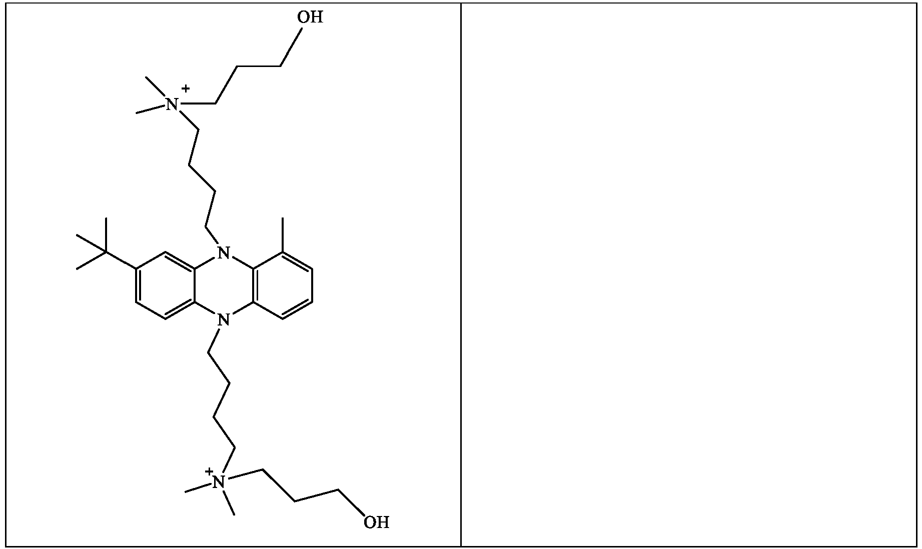

In another aspect, an electrochromic device includes an anodic material coated on to the surface of a first conductive substrate and covalently crosslinked, and a cathodic material coated on to the surface of a second conductive substrate and covalently crosslinked. The electrochromic device exhibits a high transmission state at short circuit and a low transmission state upon application of an electric potential, where the high transmission state is at least 4 times greater than the low transmission state; and the electrochromic device is configured to maintain a transmission percentage within 5% of the low transmission state for at least 8 hours at 20°C at open circuit after application of a potential sufficient to reach to the low transmissions state. In some embodiments, the electrochromic device is configured to maintain a transmission percentage within 5% of the low transmission state for at least 8, 10, 15, 20, 24, or 48 hours at 20°C at open circuit after application of a potential sufficient to reach to the low transmissions state. In some embodiments, the device is configured to maintain a transmission percentage within 10% of the low transmission state for at least 2 days, 3 days, 4 days, or 5 days at 20°C at open circuit after application of a potential sufficient to reach to the low transmissions state. In some embodiments, the device is configured to maintain a transmission percentage within 10% of the low transmission state from 8 hours to 10 days at 20°C at open circuit after application of a potential sufficient to reach to the low transmissions state. The low transmission state may range from about 0.001% to about 30%. The high transmission state range from about 50% to about 95%. In some embodiments, after 4000 cycles from the high transmission state to the low transmission state, a high transmission value does not vary by more than 5% from an initial high transmission value. As used herein the initial high transmission value is the state of the device prior to the application of an electric potential after device fabrication. In some embodiments, after 4000 cycles from high transmission to low transmission, the low transmission value does not vary by more than 5% from an initial low transmission value. As used herein, the initial low transmission value is the low transmission value achieved upon the first charging of the device at a full voltage application.

In any of the above aspects, the cathodic material may be a viologen, a low-dimerizing viologen or a non-dimerizing viologen. The term low-dimerizing viologen is applied to some viologens that show dimerization characteristics to a lesser extent that dimerizing viologens. The viologen material may be sequestered within the polymer matrix by being physically trapped within, or the viologen material may be functionalized such that it is amenable to being polymerized or reacted with the polymer to be covalently bonded to the polymer. Illustrative viologens include, but are not limited to, methyl viologen, octyl viologen, benzyl viologen, polymeric viologens, and the viologens described in US 4 902 108 A; US 6 188 505 B1; US 5 998 617 A; US 10 464 900 B2; and US 6 710 906 B2. Other viologens may include those of Formula (I), (III), or (IV): 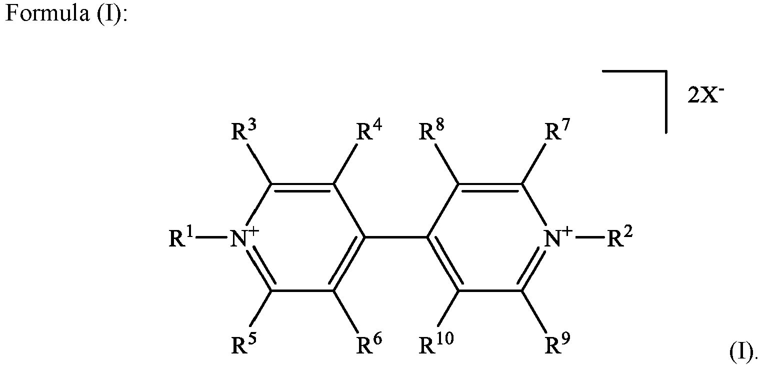 In Formula I, R¹ and R² are individually alkyl, siloxy alkyl, hydroxyalkyl, alkenyl, or aralkyl; R⁴, R⁶, R⁸, and R¹⁰ are individually H, OH, F, Cl, Br, I, CN, NO₂, alkyl, alkoxy, or aryl; R³, R⁵, R⁷, and R⁹ are individually H, OH, F, Cl, Br, I, CN, NO₂, alkyl, alkoxy, or aryl, and X is an anion. However, Formula (I) may be subject to the proviso that R³ and R⁵, or R⁷ and R⁹, or R³, R⁵, R⁷, and R⁹ are individually secondary alkyl, tertiary alkyl, or aryl. 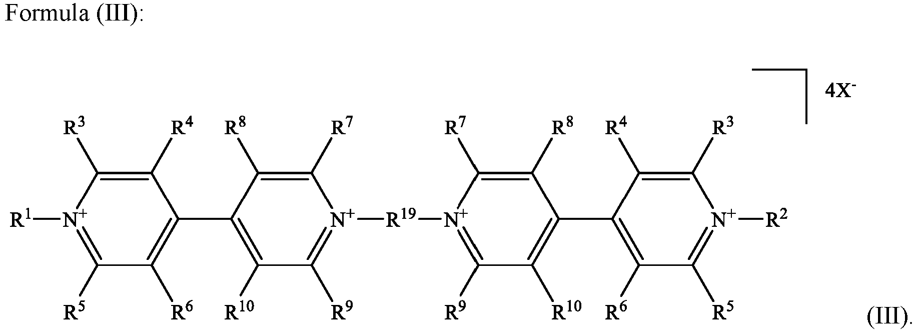 In Formula (III), R¹ and R² are individually alkyl, siloxyalkyl, hydroxyalkyl, alkenyl, or aralkyl; R⁴, R⁶, R⁸, R¹⁰ are individually H, OH, F, Cl, Br, I, CN, NO₂, alkyl, alkoxy, or aryl; R³, R⁵, R⁷, and R⁹ are individually H, OH, F, Cl, Br, I, CN, NO₂, alkyl, alkoxy or aryl; R¹⁹ is (CH₂)_{n'} or arylene, and n' is from 1 to 12; X is an anion; and wherein R³ and R⁵, or R⁷ and R⁹ are individually secondary alkyl, tertiary alkyl, or aryl. 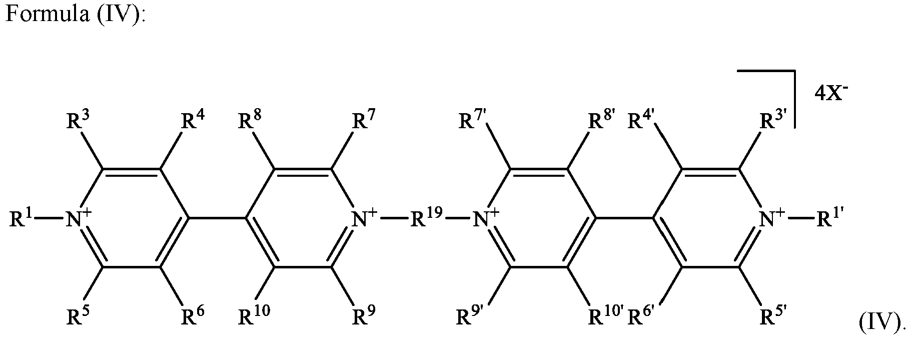 In Formula (IV), R¹ and R^{1'} are individually alkyl, siloxyalkyl, hydroxyalkyl, alkenyl, or aralkyl; R⁴, R⁶, R⁸, R¹⁰, R^{4'}, R^{6'}, R^{8'}, and R^{10'} are individually H, OH, F, Cl, Br, I, CN, NO₂, alkyl, alkoxy, or aryl; R⁷, R⁹, R^{7'}, and R^{9'} are individually H, OH, F, Cl, Br, I, CN, NO₂, alkyl, alkoxy, or aryl; R¹⁹ is (CH₂)_{n'} or arylene, and n' is from 1 to 12; X is an anion; and either R³, R⁵, R^{3'}, and R^{5'} are individually secondary alkyl, tertiary alkyl, or aryl; or R⁷, R⁹, R^{7'}, and R^{9'} are individually secondary alkyl, tertiary alkyl, or aryl. For any of the viologens described, the counterion (anion) may be any of a halide, a borate, a fluoroborate, a tetraaryl borate, a hexafluoro metal or metalloid, a sulfate, a sulfonate, a sulfonamide, a carboxylate, a perchlorate, a tetrachloroferrate, or the like, or mixtures of any two or more thereof. Illustrative counterions/anions include, but are not limited to: F⁻, Cl⁻, Br⁻, I⁻, BF₄⁻, PF₆⁻, SbF₆⁻, AsF₆⁻, ClO₄⁻, SO₃CF₃⁻, N(CF₃SO₂)₂⁻,C(CF₃SO₂)₃⁻, N(SO₂C₂F₅)₂⁻, or BAr₄⁻, wherein Ar is a aryl or a fluorinated aryl or a bis(trifluoromethyl) aryl group. In some embodiments, X is a tetrafluoroborate or a bis(trifluoromethylsulfonyl) imide anion.

In any of the above aspects, the anodic material may be any one or more of the phenazines compounds described herein covalently attached to, or confined within, the second polymer matrix, the second polymer matrix configured to prevent or minimize substantial diffusion of the anodic material in the activated state. As with the viologen, the anodic material may be sequestered within the polymer matrix by being physically trapped within, or the anodic material may be functionalized such that it is amenable to being polymerized or reacted with the polymer to be covalently bonded to the polymer.

Further examples of anodic and cathodic materials may be found in US 4 902 108 A; US 5 294 376 A; US 5 998 617 A; US 6 193 912 B1; US 10 971 718 B2; and US 8 228 590 B2.

Where the devices include an electrolyte, the electrolyte may include a solvent and a salt. The salt may be a metal salt or an ammonium salt. Illustrative solvents for use in the electrolyte may include, but are not limited to, 3-methylsulfolane, dimethyl sulfoxide, dimethyl formamide, tetraglyme and other polyethers; alcohols such as ethoxyethanol; nitriles, such as acetonitrile, glutaronitrile, 3-hydroxypropionitrile, and 2-methylglutaronitrile; ketones including 2-acetylbutyrolactone, and cyclopentanone; cyclic esters including beta-propiolactone, gamma-butyrolactone, and gamma-valerolactone; propylene carbonate (PC), ethylene carbonate; and homogenous mixtures of the same. While specific solvents have been disclosed as being associated with the electrolyte, numerous other solvents that would be known to those having ordinary skill in the art having the present disclosure before them are likewise contemplated for use. Illustrative salts include, but are not limited to, metal or ammonium salts, such as but not limited to Li⁺, Na⁺, K⁺, NR'₄⁺, where each R' is individually H, alkyl, or cycloalkyl, of the following anions F⁻, Cl, Br⁻, I⁻, BF₄⁻, PF₆⁻, SbF₆⁻, AsF₆⁻, ClO₄⁻, SO₃CF₃⁻, N(CF₃SO₂)₂⁻,C(CF₃SO₂)₃⁻, N(SO₂C₂F₅)₂⁻, Al(OC(CF₃)₃)₄⁻, or BAr₄⁻, wherein Ar is an aryl or fluorinated aryl group such as, but not limited to, C₆H₅, 3,5-(CF₃)₂C₆H₃]₄, or C₆F₅. Further examples of polymer electrolyte materials used in electrochromic devices can be found in US 6 635 194 B2 and US 5 940 201 A.

With regard to the substrates and conductive coatings on the substrates, those typically used in solution-based electrochromic devices may be used. For example, the one or both substrates may be glass, metal, plastic, or ceramic. The conductive coating on one or more of the substrates may be transparent or opaque depending upon the intended use of the device. For example, where the device is a window, both coatings should be substantially transparent, and where the device is a mirror at least one coating is transparent. Illustrative, transparent conductive materials include, but are not limited to, fluorine doped tin oxide (FTO), indium/tin oxide (ITO), doped zinc oxide, indium zinc oxide, metal oxide/Ag/metal oxide, silver nano-wire coatings, carbon nanotubes, graphene coatings, wire grids, conductive polymers such as, but not limited to, poly(3,4-ethylenedioxythiophene) (PEDOT). Non-transparent conductive coatings include metal coatings such as rhodium, chromium, nickel, silver, gold, and other metals, or mixtures of any two or more thereof.

The present invention, thus generally described, will be understood more readily by reference to the following examples, which are provided by way of illustration and are not intended to be limiting of the present invention.

### EXAMPLES

**Example 1.** Synthesis of: N-butyl-4-(2,7-di-tert-butyl-10-(4-((3-hydroxypropyl)dimethylammonio)butyl)phenazin-5(10H)-yl)-N,N-dimethylbutan-1-aminium hexafluorophosphate.

The four step synthesis of the title compound is as follows:
**Step 1:** 2,7(8)-di-tert-butyl-5,10-dihydrophenazine (as a mixture of two isomers) 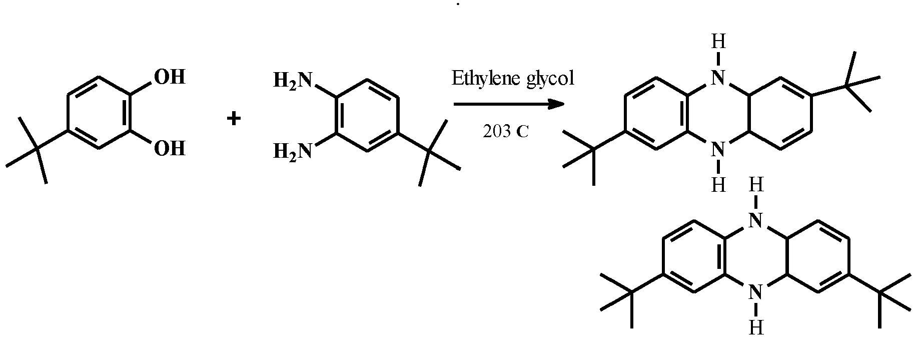
**Step** 2: 4-bromo-N-(3-hydroxypropyl)-N,N-dimethylbutan-1-aminium bromide 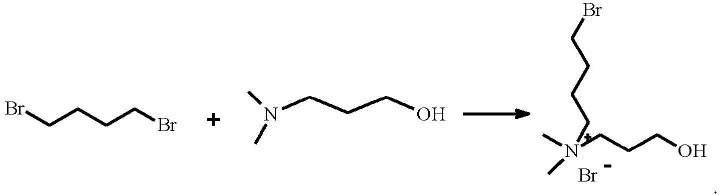
**Step 3:** N-butyl-4-(2,7-di-tert-butyl-10-(4-((3-hydroxypropyl)dimethylammonio) butyl)phenazin-5(10H)-yl)-N,N-dimethylbutan-1-aminium bromide 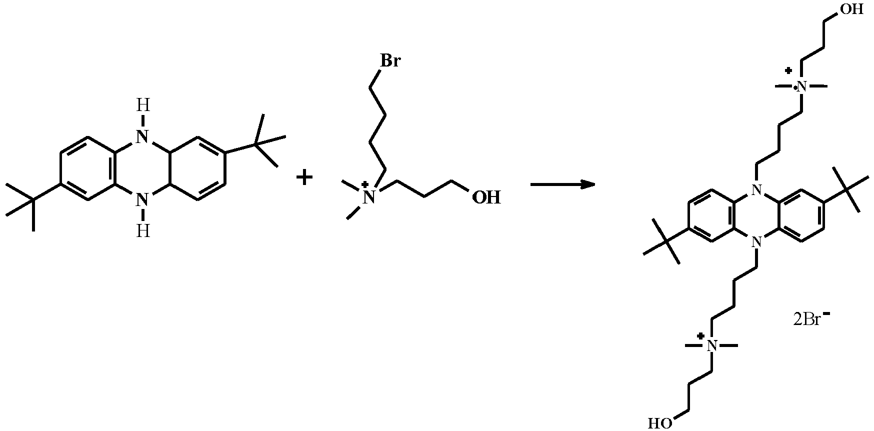
**Step 4:** Metathesis to PF₆ salt

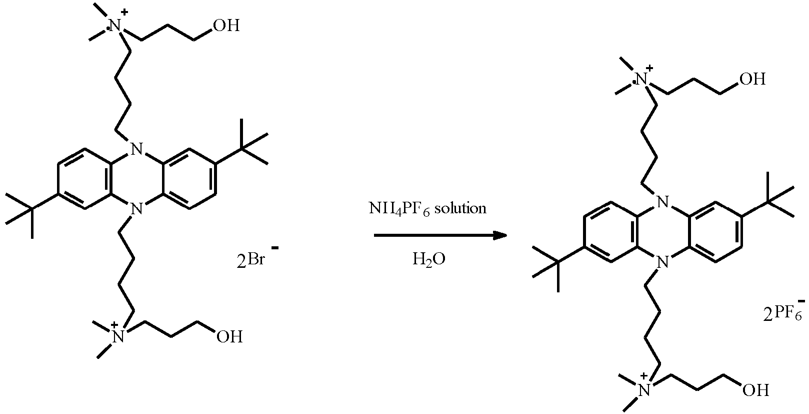

Step 1: 400.0 g of 4-(tert-butyl)benzene-1,2-diamine, 992 g of 4-(tert-butyl)benzene-1,2-diol, and 1330 mL of ethylene glycol were added to a three neck 5L round bottom flask equipped with a dean-stark condenser, a mechanical stirrer, and a thermometer. The mixture was heated to 203°C for 1 week under a nitrogen atmosphere. The reaction mixture was then cooled to 60°C. The solid was filtered under inert condition to prevent oxidation. The solid was then washed with a mixture of 1:1 acetone and water (4L). The solid was recrystallized by first partially dissolving in 1L acetone and then a solution of sodium dithionite in water was added (160g dissolved in 2L water). Finally, the solution was cooled to room temperature and the solid was filtered under inert conditions. In vacuo removal of the solvent provided 2,7-di-tert-butyl-5,10-dihydrophenazine in 516 g (72 % yield). GC analysis showed 99.8% pure product.

Step 2: To a 12-liter round bottom flask was charged 1,4-dibromobutane (2260 g) and acetone (6L). The flask was cooled to 5-7°C. The dimethyl amino propanol (1200 g) was slowly added via addition funnel. The reaction was slightly exothermic. Once the addition was complete, the temperature was allowed to rise to room temperature. After stirring at room temperature for 48 hours, the product precipicated out as a white crystal. The solid product was filtered and washed with acetone. Vacuum drying provided 3112 g of the desired product (yield 93%).

Step 3: 240 g of 2,7-di-tert-butyl-5,10-dihydrophenazine, 1154 g of 4-bromo-N-(3-hydroxypropyl)-N,N-dimethylbutan-1-aminium bromide, 280 g of sodium dithionite, 375 g of sodium carbonate, 30g methyl tributyl ammonium chloride, 30 ml water and 7400 mL of acetonitrile were added to a 12 L round bottom flask. The mixture was heated to reflux under a nitrogen atmosphere for 48 hours, where HPLC analysis showed no dihydrophenazine was present. 3 L of acetonitrile was then distilled off and the reaction mixture was allowed to cool down to room temperature, where the reaction solids were then filtered out and washed with 2.5 L acetone. The solid was redissolved in 1.3 L water, heated, and residual acetone was distilled off at 70°C. 2 L Ethanol was added and the reaction mixture was cooled to room temperature. The solid was filtered and washed with ethanol. The solid was discarded and the filtrate containing the product was concentrated to oil in vacuo. The oil was added to 6 L hot acetone. Upon cooling, the filtrate was decanted, and the gummy solid was washed with additional acetone. The gummy solid was N-butyl-4-(2,7-di-tert-butyl-10-(4-((3-hydroxypropyl)dimethylammonio)butyl)phenazin-5(10H)-yl)-N,N-dimethylbutan-1-aminium bromide and was taken directly to make the PF₆ salt. The product was 99% pure by HPLC and mass ES analysis.
Step 4: Synthesis of: N-butyl-4-(2,7-di-tert-butyl-10-(4-((3-hydroxypropyl)dimethylammonio)butyl)phenazin-5(10H)-yl)-N,N-dimethylbutan-1-aminium hexafluorophosphate.

First metathesis: The gummy solid of N-butyl-4-(2,7-di-tert-butyl-10-(4-((3-hydroxypropyl)dimethylammonio)butyl)phenazin-5(10H)-yl)-N,N-dimethylbutan-1-aminium bromide was dissolved in a 900 mL of water and 100 ml triethyl amine mixture by heat to 50°C. 2500 mL of 30% NH₄PF₆ solution in water was added, and the reaction was allowed to stir at 50°C for 5 hours. The reaction was cooled to precipitate N-butyl-4-(2,7-di-tert-butyl-10-(4-((3-hydroxypropyl)dimethylammonio)butyl)phenazin-5(10H)-yl)-N,N-dimethylbutan-1-aminium hexafluorophosphate as a grey solid in 720g of wet solid.

The second metathesis was repeated by dissolving the solid in 350 ml acetonitrile, 1000 ml water, 150 ml triethylamine and 1000 ml ethanol. The reaction mixture was heated to 65°C and 1700 ml 30% NH₄PF₆ solution was added. After continued heating for 3 hours, 1000 ml water was added. 150 ml acetonitrile was distilled off, and the reaction was cooled to room temperature. Upon cooling, the product solidified. The filtrate was decanted, and the product washed with 3L cold water to give a gummy solid. The solid was dissolved in 1000 ml acetonitrile. The acetonitrile and residual water were completely removed under vacuum to give a brown concentrate. To the brown concentrate was added to a hot solution mixture of 4000 ml ethanol, 100 ml triethylamine, and 10 ml anhydrous hydrazine. After continued heating at 60°C for 15 minutes, the reaction was cooled to 40°C and 2000 ml hexane was added. The reaction was cooled to room temperature and then at 0-5°C for 4 hours. The solid was filtered under inert conditions and washed with 1000 ml cold ethanol and then 1000 ml 1: 1 mixture of ethanol and hexane. The solid was vacuum dried to give 494 g (yield 64%) of a off white solid. The product is 99.5% pure by HPLC and mass spectroscopy.

### Example 2. Synthesis of: N-butyl-4-(2,7-di-tert-butyl-10-(4-((3-hydroxypropyl)dimethylammonio)butyl)phenazin-5(10H)-yl)-N,N-dimethylbutan-1-aminium triflate:

50 g of N-butyl-4-(2,7-di-tert-butyl-10-(4-((3-hydroxypropyl)dimethylammonio) butyl)phenazin-5(10H)-yl)-N,N-dimethylbutan-1-aminium bromide was dissolved in 85 mL of hot water and 250 mL of 30% sodium triflate was added. The reaction mixture was heated to 60°C for 4 hours. 200 ml water was added followed by cooling to room temperature. The product was filtered and washed with 300 ml water under inert conditions.

The second metathesis was done by dissolving the above solid in 150 ml hot water. 20 ml triethylamine was added. The reaction was allowed to stir at 50°C for 5 hours. The reaction was cooled and filtered to give N-butyl-4-(2,7-di-tert-butyl-10-(4-((3-hydroxypropyl)dimethylammonio)butyl)phenazin-5(10H)-yl)-N,N-dimethylbutan-1-aminium triflate as a white solid in 50 g (82% yield) after vacuum drying. The product was 99.9% pure by HPLC and mass spectroscopy.

### Example 3. Formulation and Process for Preparing Phenazine-Based Devices

In this Example, Device A was made with the following compound:

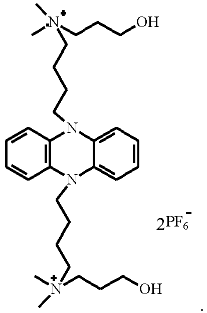

In this Example, Device B was made with the following compound:

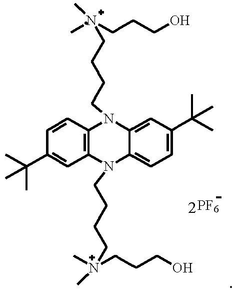

Device A was prepared by coating an indium tin oxide ( ITO) coated piece of 7.62 mm (3-inch) by 7.62 mm (3-inch) glass (2.2 mm thickness) with a mixture made by dissolving 0.3518 grams of bis N,N'-(6-hydroxyhexyl) viologen bis [bis trifluoromethanesulfonyl imide] (NTF) , 0.0149 grams of octamethyl bis(6-hydroxyhexyl) ferrocenium bis(tetrafluoroborate), 0.0006 grams of surfactant (TEGO Glide 410), and 0.1433 grams of HDT-Isocyanurate (Desmodur N3300, Covestro) in a 3 gram solution of propylene carbonate (PC) and 3-methoxypropionitrile (MPN) (35% wt PC, 65% wt MPN), plus 100 ppm of dibutyltin diacetate (DBTDA) catalyst. The mixture was coated directly on the ITO coating with a # 16 Mayer rod to control thickness.

Also for device A, second piece of 7.62 mm (3-inch) by 7.62 mm (3-inch) ITO coated glass (2.2 mm thickness) was coated with a solution made by dissolving 0.3969 grams of 5,10-bis(n-butyl-N,N-dimethylammonium n-propanol) phenazine triflate, 0.0166 grams of octamethyl bis(6-hydroxyhexyl) ferrocene, 0.0006 grams of surfactant (TEGO Glide 410) and 0.1866 grams of HDT-Isocyanurate (Desmodur N3300, Covestro) in a 3 gram solution in propylene carbonate (PC) and 3-methoxypropionitrile (MPN) (35 % wt PC, 65 % wt MPN), plus 100 ppm of DBTDA catalyst. The second film was made by coating the solution with a # 8 Mayer rod to control thickness.

Device B was prepared by coating an indium tin oxide (ITO) coated piece of 7.62 mm (3-inch) by 7.62 mm (3-inch) glass (2.2 mm thickness) with a solution made by dissolving 0.3588 grams of bis (6 - hydroxyhexyl) viologen bis [bistrifluoromethane sulfonyl imide] (NTF) , 0.0149 grams of octamethyl dihexanol ferrocenium bis(tetrafluoroborate), 0.0006 grams of surfactant (TEGO Glide 410), and 0.1433 grams of HDT-Isocyanurate (Desmodur N3300, Covestro) in a 3 gram solution in propylene carbonate (PC) and 3-methoxypropionitrile (MPN) (35% PC, 65% MPN) plus 100 ppm of dibutyl tin diacetate (DBTDA) catalyst. This film was made by coating the solution with a # 16 Mayer rod to control the thickness.

Also, for device B, a second piece of 7.62 mm (3-inch) by 7.62 mm (3-inch) ITO coated glass (2.2 mm thickness) was coated with a solution made by dissolving 0.4112 grams of N-butyl-4-(2,7-di-tert-butyl-10-(4-((3-hydroxypropyl)dimethylammonio)butyl)phenazin-5(10H)-yl)-N,N-dimethylbutan-1-aminium bis(hexafluorophosphate), 0.0152 grams of octamethyl bis(6-hydroxyhexyl) ferrocene, 0.0006 grams of surfactant (TEGO Glide 410), and 0.1706 grams of HDT-Isocyanurate (Desmodur N3300, Covestro) in a 3 gram solution in propylene carbonate (PC) and 3-methoxypropionitrile (MPN) (35 % PC, 65 % MPN), plus 100ppm of DBTA, thereby forming a second film. This second film was made with a # 8 Mayer rod.

The films above were allowed to cure under a nitrogen atmosphere overnight in an oven at 60 °C. Once the films were cured, the edges of the films were removed (less than a centimeter) to accommodate the epoxy seal and electrical contact to the ITO. The first and second films on their respective ITO coated glass substrates were positioned in a space-apart relationship facing each other, and an epoxy seal was placed around the perimeter and cured to form a cell leaving offsets for attachment of electrical contacts. The distance between the two substrates was about 135 micrometers. The resulting cell was filled with a solution of 0.1 molar tetraethyl ammonium bis-trifluoromethanesulfonimidate (TEANTF) and a cross-linkable polymer matrix precursor in PC to form a gel electrolyte.

The Devices A and B with two different anodic compounds were subjected to a variety of durability tests.

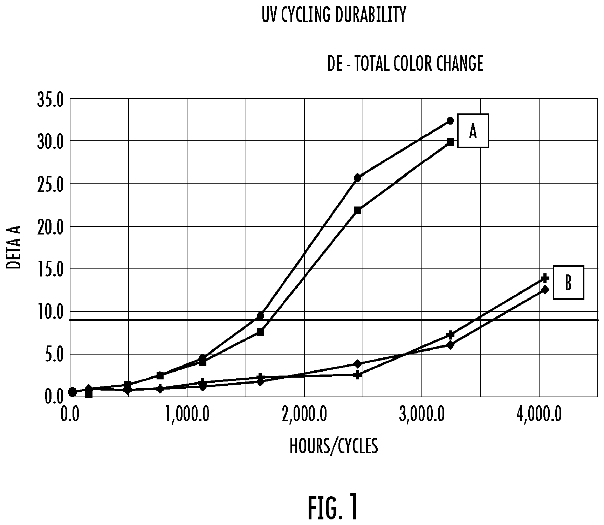

The results of the UV cycling durability test for Devices A and B are shown in FIG. 1. The devices were exposed to a Xenon lamp weatherometer (Atlas WeatherOmeter at .55W/m² at 340 nm) for 4000 hours. Devices A and B were tested behind a light attenuator that was made by laminating two sheets of Kuraray UV extra protect PVB film (Trosifol^{®}) and one sheet of 3M ^{™} Ultraclear Solar IR reflecting film between two sheets of sodalime glass, one 1.1 mm thick and one 2.2 mm thick. Cycling profile for both devices was 3-hours in the darken state, 1-hour in the clear state and repeat. Device A was cycled between clear state (0 volts) and full dark state (0.8 volts) while Device B was cycled between clear state (0 volts) and full dark state (0.7 volts). Delta E in UV cycling for Device B after 4000 hours was only 13.9 while the Device A showed significant color change with delta E 30.

Device B made with phenazine compound with tert butyl groups showed significant improvement in UV cycling durability as shown in FIG. 1.

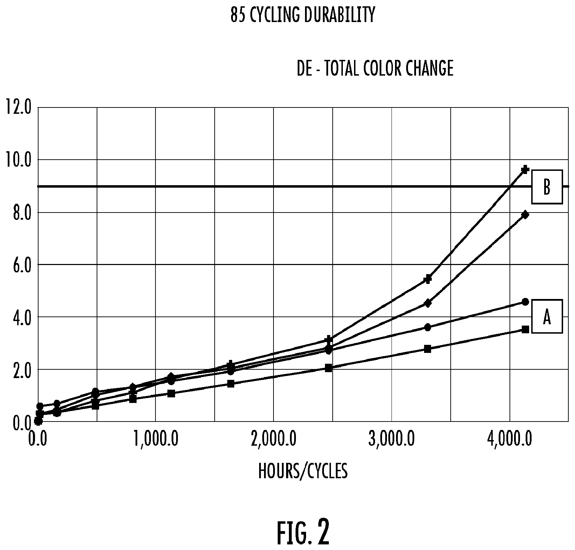

The results of the 85°C cycling durability testing for Devices A and B are shown in FIG. 2. Cycling profile for both devices was 3-hours in the darken state, 1-hour in the clear state and repeat. Device A was cycled between clear state (0 volts) and full dark state (0.8 volts) while Device B was cycled between clear state (0 volts) and full dark state (0.7 volts).

## Claims

1. A compound represented by: 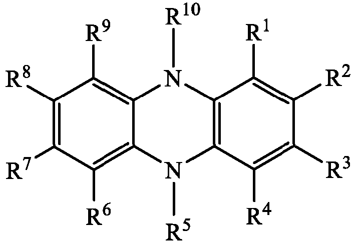 wherein:
R¹, R², R³, R⁴, R⁶, R⁷, R⁸, and R⁹ are each independently H, F, Cl, Br, I, CF₃, CN, OR¹¹, SR¹¹, NO₂, alkyl, alkoxy, aryl, heteroaryl, amino, or ammonium, or any two adjacent groups of R³, R⁴, R⁸, and R⁹ may join to form a monocyclic, polycyclic, or heterocyclic group;
each R¹¹ is independently H or alkyl;
R⁵ and R¹⁰ are independently represented by:
-L₁-A-L₂-OR¹⁴;
each L₁ is independently an C₁-C₈ alkylene group;
each L₂ is independently an C₁-C₈ alkylene group;
each R¹⁴ is H or alkyl;
A is an ammonium group represented by -N(R¹⁵)₂⁺-;
each R¹⁵ is alkyl or alkylhydroxy; and
at least one of R², R³, R⁷, and R⁸ is a sterically hindered group.

2. The compound of claim 1, wherein the sterically hindered group is selected from tertiary butyl, isobutyl, isopropyl, 2-ethyl butyl, 2-ethyl hexyl, sec-butyl, isopentyl, and neopentyl.

3. The compound of claim 1, wherein R¹, R³, R⁴, R⁶, R⁸, and R⁹ are each H.

4. The compound of claim 1, wherein R¹, R³, R⁴, R⁶, R⁷, and R⁹ are each H.

5. The compound of claim 1, wherein at least one of R², R³, R⁷, and R⁸ are independently C₂-C₈ alkyl.

6. The compound of claim 1, wherein R² and R⁷, R² and R⁸, R³ and R⁸, or R³ and R⁷ are each independently C₂-C₈ alkyl.

7. The compound of claim 1, wherein at least one of R², R³, R⁷, and R⁸ are independently aryl.

8. The compound of claim 1, wherein at least one of R⁵ and R¹⁰ are independently represented by:
-L₁-A-L₂-OR¹⁴;
each L₁ is independently an C₁, C₂, C₃, C₄, C₅, C₆, C₇, or C₈ alkylene group;
each L₂ is independently an C₁, C₂, C₃, C₄, C₅, C₆, C₇, or C₈ alkylene group;
each R¹⁴ is H or alkyl;
A is an ammonium group represented by -N(R¹⁵)₂⁺-; and
each R¹⁵ is C₁-C₈ alkyl or C₁-C₈ alkylhydroxyl.

9. The compound of claim 8, wherein:
each L₁ is independently an C₂ or C₄ alkylene group;
each L₂ is independently an C₃ alkylene group;
each R¹⁴ is H;
A is an ammonium group represented by -N(R¹⁵)₂⁺-; and
each R¹⁵ is C₁ alkyl or C₂ alkylhydroxyl.

10. The compound of claim 1, wherein at least one of R⁵ and R¹⁰ are independently represented by: 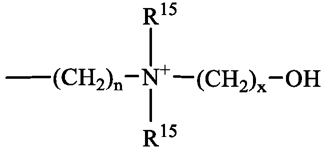
n is 1, 2, 3, 4, 5, 6, 7, or 8;
x is 1, 2, 3, 4, 5, 6, 7, or 8; and
each R¹⁵ is independently C₁-C₈ alkyl or C₁-C₈ alkylhydroxyl.

11. The compound of claim 10,
n is 2 or 4;
x is 3; and
each R¹⁵ is independently C₁ alkyl or C₂ alkylhydroxyl.

12. The compound of claim 1, wherein the compound is ammonium salt with an anion comprising F⁻, Cl⁻, Br⁻, I⁻, BF₄⁻, PF₆⁻, SbF₆⁻, AsF₆⁻, ClO₄⁻, SO₃CF₃⁻, N(CF₃SO₂)₂⁻, C(CF₃SO₂)₃⁻, N(SO₂C₂F₅)₂⁻, Al(OC(CF₃)₃)₄⁻, BAr₄⁻, or mixtures of anions thereof, wherein Ar is an aryl or fluorinated aryl group.

13. The compound of claim 1, wherein the compound is: 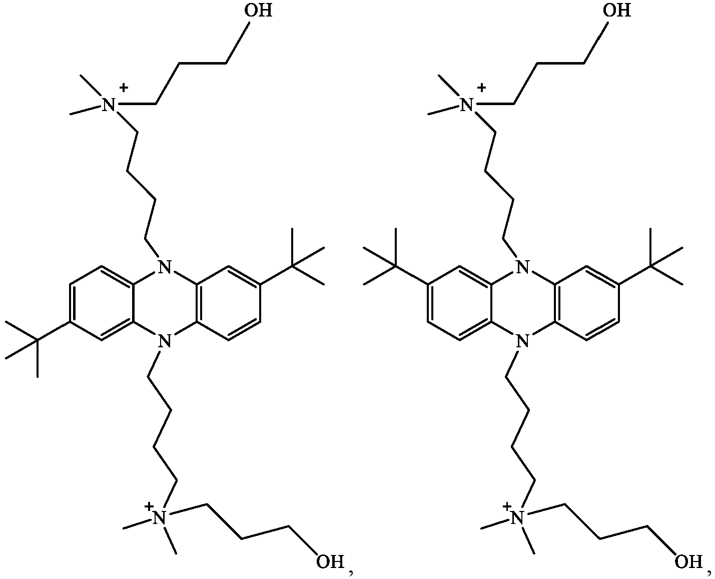 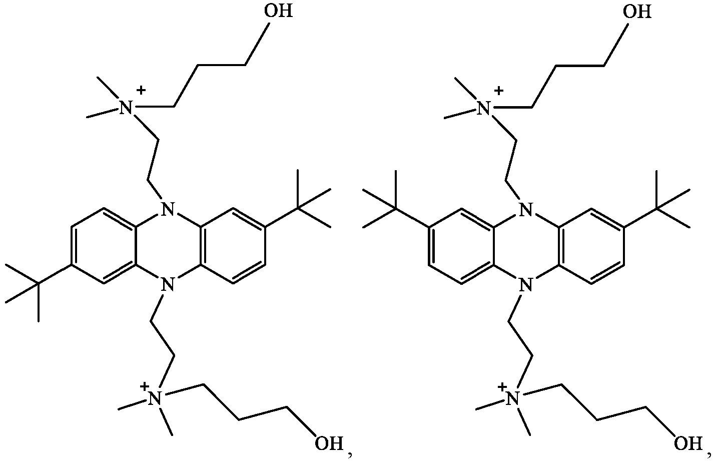 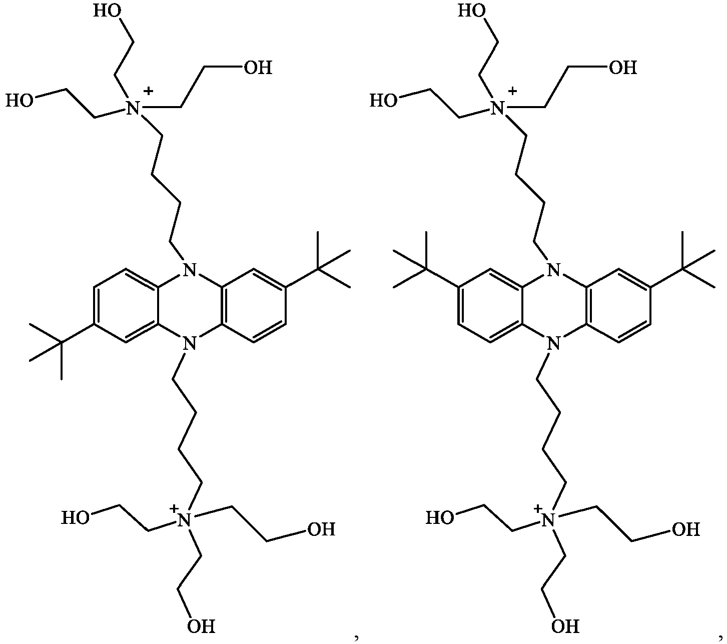 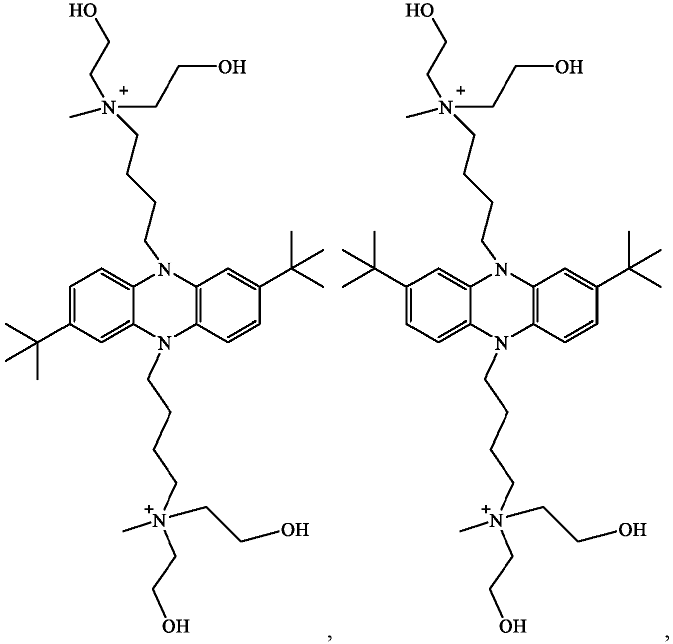 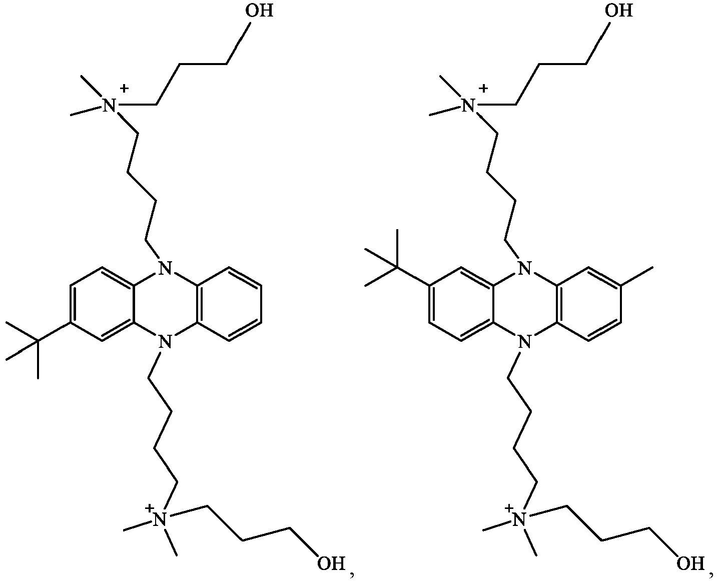 or

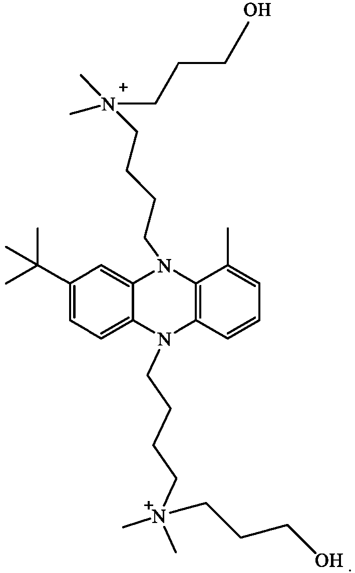

## Patentansprüche

1. Verbindung, dargestellt durch: 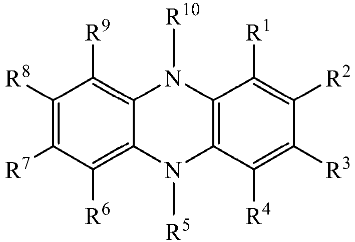 worin:
R¹, R², R³, R⁴, R⁶, R⁷, R⁸ und R⁹ jeweils unabhängig H, F, Cl, Br, I, CF₃, CN, OR¹¹, SR¹¹, NO₂, Alkyl, Alkoxy, Aryl, Heteroaryl, Amino oder Ammonium sind, oder zwei beliebige benachbarte Gruppen von R³, R⁴, R⁸ und R⁹ sich zu einer monocyclischen, polycyclischen oder heterocyclischen Gruppe verbinden können;
jedes R¹¹ unabhängig H oder Alkyl ist;
R⁵ und R¹⁰ unabhängig voneinander dargestellt werden durch:
-L₁-A-L₂-OR¹⁴;
jedes L₁ unabhängig eine C₁-C₈ Alkylengruppe ist;
jedes L₂ unabhängig eine C₁-C₈ Alkylengruppe ist;
jedes R¹⁴ H oder Alkyl ist;
A eine Ammoniumgruppe ist, dargestellt durch -N(R¹⁵)₂⁺-;
jedes R¹⁵ Alkyl oder Alkylhydroxy ist; und
mindestens eine der Gruppen R², R³, R⁷ und R⁸ sterisch gehindert ist.

2. Verbindung nach Anspruch 1, wobei die sterisch gehinderte Gruppe ausgewählt ist aus tertiärem Butyl, Isobutyl, Isopropyl, 2-Ethylbutyl, 2-Ethylhexyl, sec-Butyl, Isopentyl und Neopentyl.

3. Verbindung nach Anspruch 1, wobei R¹, R³, R⁴, R⁶, R⁸ und R⁹ jeweils H sind.

4. Verbindung nach Anspruch 1, wobei R¹, R³, R⁴, R⁶, R⁷ und R⁹ jeweils H sind.

5. Verbindung nach Anspruch 1, wobei mindestens einer der Reste R², R³, R⁷ und R⁸ unabhängig voneinander ein C₂-C₈ Alkylrest ist.

6. Verbindung nach Anspruch 1, wobei R² und R⁷ , R² und R⁸ , R³ und R⁸ oder R³ und R⁷ jeweils unabhängig voneinander C₂-C₈ Alkylreste sind.

7. Verbindung nach Anspruch 1, wobei mindestens einer der Reste R², R³, R⁷ und R⁸ unabhängig voneinander Arylreste sind.

8. Verbindung nach Anspruch 1, wobei mindestens eine der Komponenten R⁵ und R¹⁰ unabhängig dargestellt werden durch:
-L₁-A-L₂-OR¹⁴;
jedes L₁ unabhängig eine C₁, C₂, C₃, C₄, C₅, C₆, C₇ oder C₈ Alkylengruppe ist;
jedes L₂ unabhängig eine C₁, C₂, C₃, C₄, C₅, C₆, C₇ oder C₈ Alkylengruppe ist;
jedes R¹⁴ H oder Alkyl ist;
A eine Ammoniumgruppe ist, dargestellt durch -N(R¹⁵)₂⁺-; und
jedes R¹⁵ ein C₁-C₈ Alkylrest oder ein C₁-C₈ Alkylhydroxylrest ist.

9. Verbindung nach Anspruch 8, wobei:
jedes L₁ unabhängig eine C₂ oder C₄ Alkylengruppe ist;
jedes L₂ unabhängig eine C₃ Alkylengruppe ist;
jedes R¹⁴ H ist;
A eine Ammoniumgruppe ist, dargestellt durch -N(R¹⁵)₂⁺-; und
jedes R¹⁵ ein C₁ Alkylrest oder ein C₂ Alkylhydroxylrest ist.

10. Verbindung nach Anspruch 1, wobei mindestens eines von R⁵ und R¹⁰ unabhängig voneinander dargestellt wird durch: 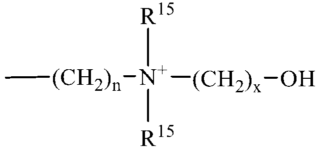
n 1, 2, 3, 4, 5, 6, 7 oder 8 ist;
x 1, 2, 3, 4, 5, 6, 7 oder 8 ist; und
jedes R¹⁵ unabhängig ein C₁-C₈ Alkylrest oder ein C₁-C₈ Alkylhydroxyl ist.

11. Verbindung nach Anspruch 10,
n 2 oder 4 ist;
x 3 ist; und
jedes R¹⁵ unabhängig ein C₁ Alkylrest oder ein C₂ Alkylhydroxylrest ist.

12. Verbindung nach Anspruch 1, wobei die Verbindung ein Ammoniumsalz mit einem Anion, umfassend F⁻, Cl⁻, Br⁻, I⁻, BF₄⁻, PF₆⁻, SbF₆⁻, AsF₆⁻, ClO₄⁻, SO₃CF₃⁻, N(CF₃SO₂)₂⁻, C(CF₃SO₂)₃⁻, N(SO₂C₂F₅)₂⁻, Al(OC(CF₃)₃)₄⁻, BAr₄⁻, oder Gemische von Anionen davon, ist, wobei Ar eine Aryl- oder fluorierte Arylgruppe ist.

13. Verbindung nach Anspruch 1, wobei die Verbindung ist: 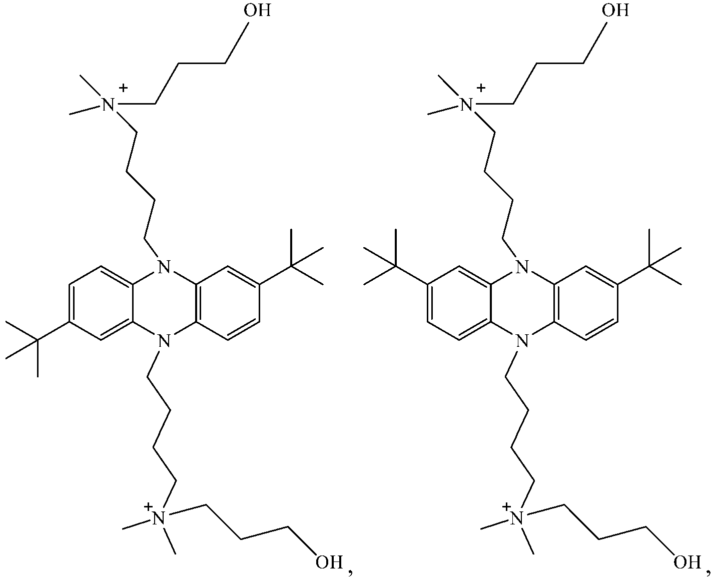 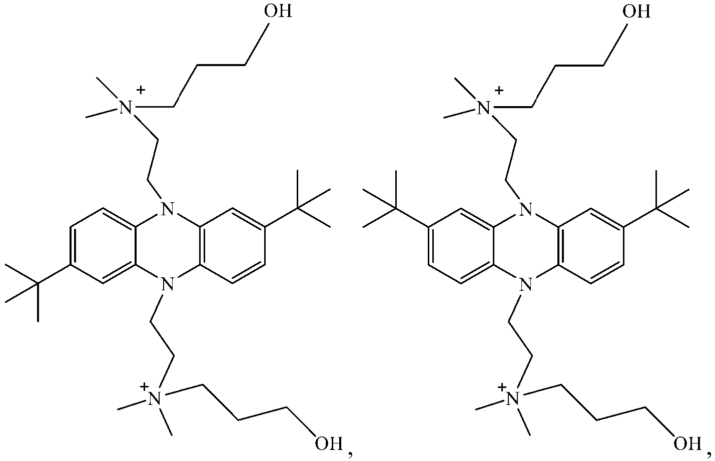 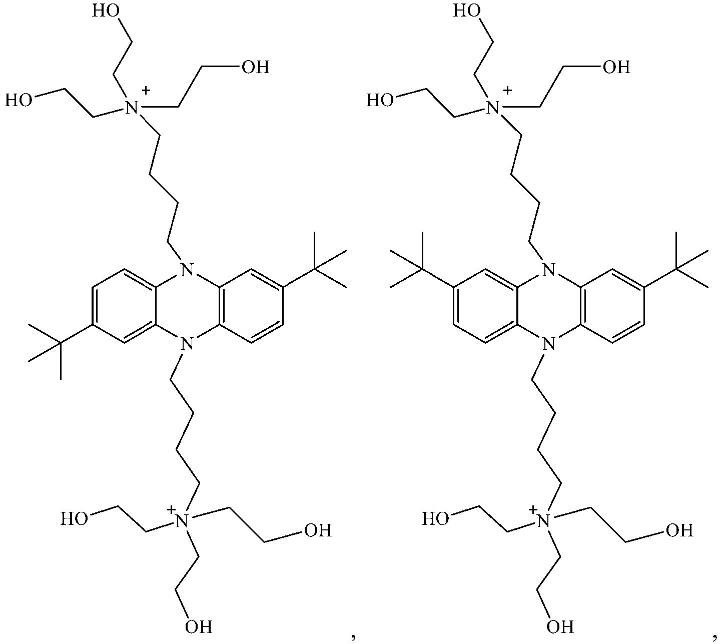 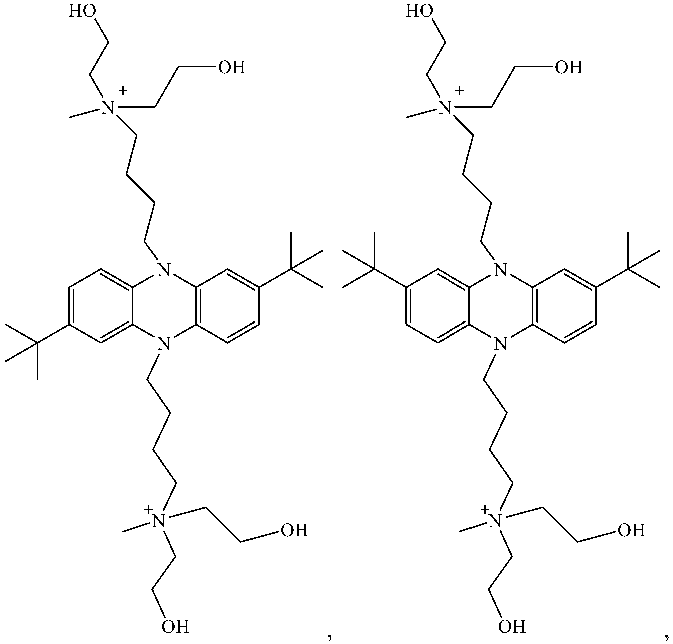 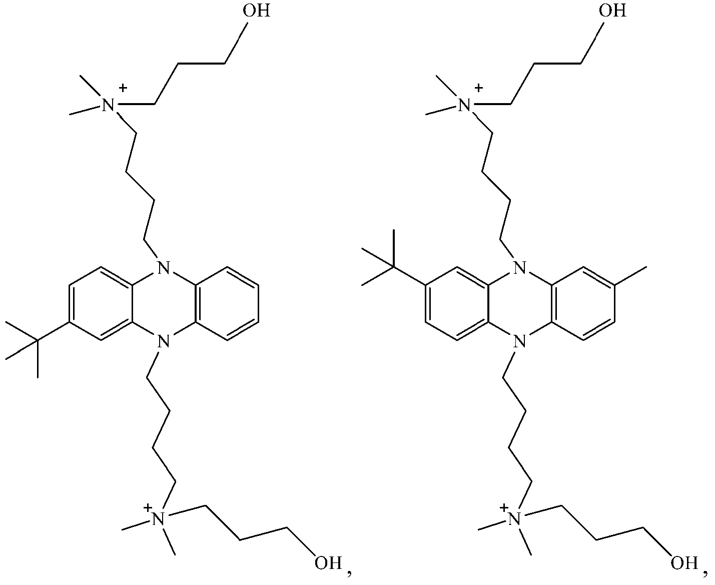 oder

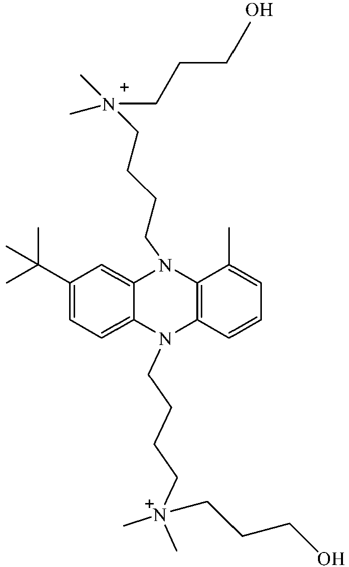

## Revendications

1. Composé représenté par : 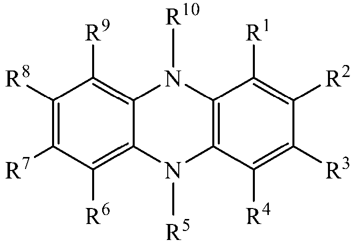 dans laquelle :
R¹, R², R³, R⁴, R⁶, R⁷, R⁸ et R⁹ sont chacun indépendamment H, F, Cl, Br, I, CF₃, CN, OR¹¹, SR¹¹, NO₂, alkyle, alcoxy, aryle, hétéroaryle, amino ou ammonium, ou deux groupes adjacents quelconques parmi R³, R⁴, R⁸ et R⁹ peuvent se lier pour former un groupe monocyclique, polycyclique ou hétérocyclique ;
chaque R¹¹ est indépendamment H ou alkyle ;
R⁵ et R¹⁰ sont indépendamment représentés par :
-L₁-A-L₂-OR¹⁴ ;
chaque L₁ est indépendamment un groupe alkylène en C₁-C₃ ;
chaque L₂ est indépendamment un groupe alkylène en C₁-C₃ ;
chaque R¹⁴ est H ou alkyle ;
A est un groupe ammonium représenté par -N(R¹⁵)₂⁺- ;
chaque R¹⁵ est alkyle ou alkylhydroxy ; et
au moins un de R², R³, R⁷ et R⁸ est un groupe à encombrement stérique.

2. Composé selon la revendication 1, dans lequel le groupe à encombrement stérique est sélectionné parmi du butyle tertiaire, de l'isobutyle, de l'isopropyle, du 2-éthylbutyle, du 2-éthylhexyle, du sec-butyle, de l'isopentyle et du néopentyle.

3. Composé selon la revendication 1, dans lequel R¹, R³, R⁴, R⁶, R⁸ et R⁹ sont chacun H.

4. Composé selon la revendication 1, dans lequel R¹, R³, R⁴, R⁶, R⁷ et R⁹ sont chacun H.

5. Composé selon la revendication 1, dans lequel au moins un de R², R³, R⁷ et R⁸ sont indépendamment alkyle en C₂-C₈.

6. Composé selon la revendication 1, dans lequel R² et R⁷, R² et R⁸, R³ et R⁸ ou R³ et R⁷ sont chacun indépendamment alkyle en C₂-C₈.

7. Composé selon la revendication 1, dans lequel au moins un de R², R³, R⁷ et R⁸ sont indépendamment aryle.

8. Composé selon la revendication 1, dans lequel au moins un de R⁵ et R¹⁰ sont indépendamment représentés par :
-L₁-A-L₂-OR¹⁴ ;
chaque L₁ est indépendamment un groupe alkylène en C₁, C₂, C₃, C₄, C₅, C₆, C₇ ou C₈ ;
chaque L₂ est indépendamment un groupe alkylène en C₁, C₂, C₃, C₄, C₅, C₆, C₇ ou C₈ ;
chaque R¹⁴ est H ou alkyle ;
A est un groupe ammonium représenté par -N(R¹⁵)₂⁺- ; et
chaque R¹⁵ est alkyle en C₁-C₈ ou alkylhydroxyle en C₁-C₈.

9. Composé selon la revendication 8, dans lequel:
chaque L₁ est indépendamment un groupe alkylène en C₂ ou C₄ ;
chaque L₂ est indépendamment un groupe alkylène en C₃ ;
chaque R¹⁴ est H ;
A est un groupe ammonium représenté par -N(R¹⁵)₂⁺- ; et
chaque R¹⁵ est alkyle en C₁ ou alkylhydroxyle en C₂.

10. Composé selon la revendication 1, dans lequel au moins un R⁵ et R¹⁰ est indépendamment représenté par : 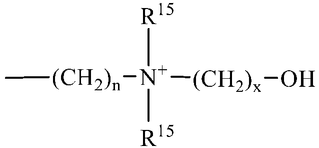
n est 1, 2, 3, 4, 5, 6, 7 ou 8 ;
x est 1, 2, 3, 4, 5, 6, 7 ou 8 ; et
chaque R¹⁵ est indépendamment alkyle en C₁-C₈ ou alkylhydroxyle en C₁-C₈.

11. Composé selon la revendication 10,
n est 2 ou 4 ;
x est 3 ; et
chaque R¹⁵ est indépendamment alkyle en C₁ ou alkylhydroxyle en C₂.

12. Composé selon la revendication 1, dans lequel le composé est un sel d'ammonium avec un anion comprenant F⁻, Cl⁻, Br⁻, I⁻, BF₄⁻, PF₆⁻, SbF₆⁻, AsF₆⁻, ClO₄⁻, SO₃CF₃⁻, N(CF₃SO₂)₂⁻, C(CF₃SO₂)₃⁻, N(SO₂C₂F₅)₂⁻, Al(OC(CF₃)₃)₄⁻, BAr₄⁻, ou des mélanges d'anions de ceux-ci, dans lequel Ar est un groupe aryle ou aryle fluoré.

13. Composé selon la revendication 1, dans lequel le composé est : 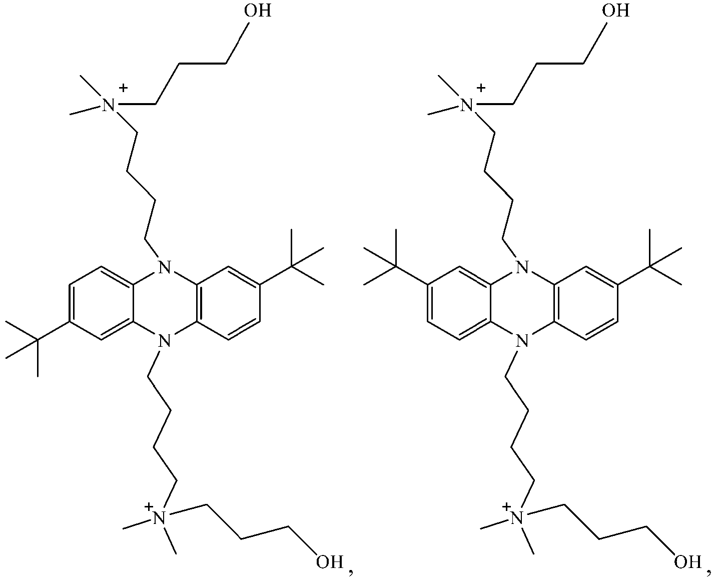 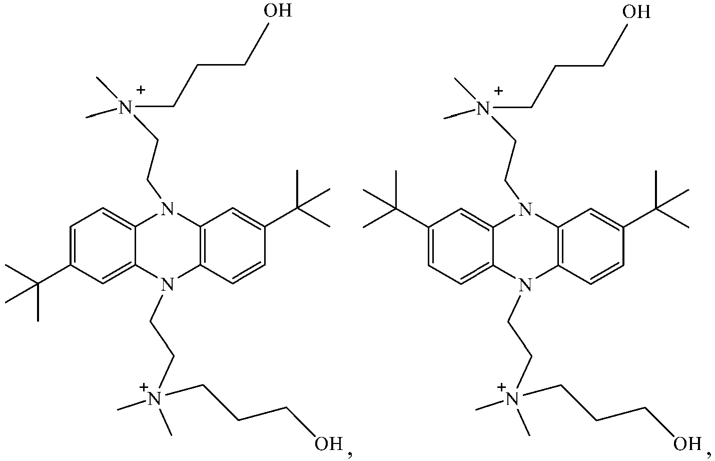 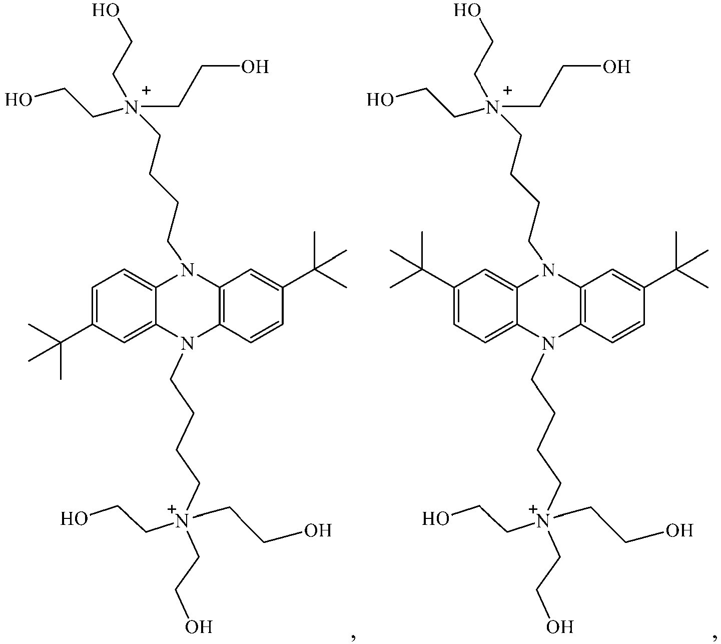 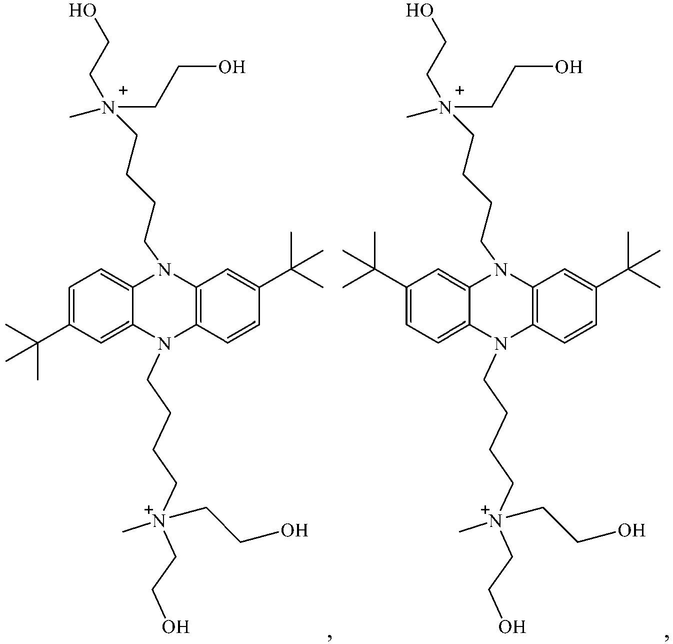 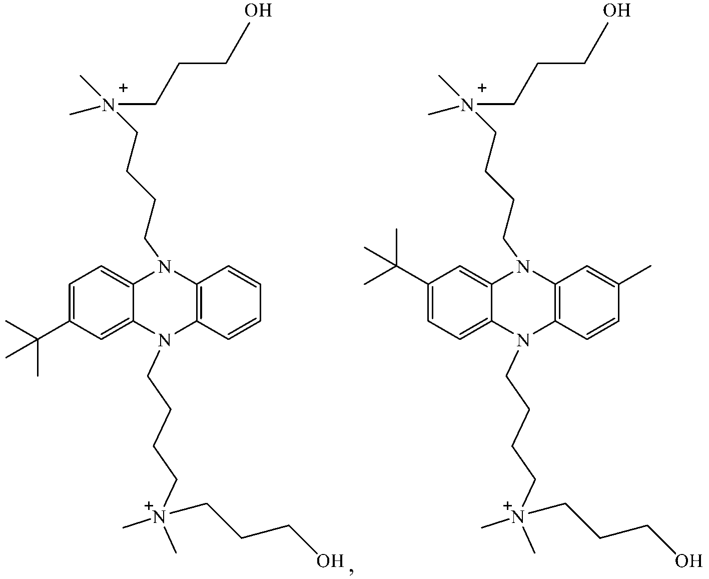 ou
